# EUROPEAN PATENT SPECIFICATION

(11) **EP 4 212 117 B1**
(45) Date of publication and mention of the grant of the patent: **20.05.2026**
(21) Application number: 21877554.2
(22) Date of filing: 04.10.2021
(51) Int. Cl.: A61B 18/12, A61B 18/14, A61B 18/00

(54) **HIGH-FREQUENCY TREATMENT DEVICE**
HOCHFREQUENZBEHANDLUNGSVORRICHTUNG
DISPOSITIF DE TRAITEMENT À HAUTE FRÉQUENCE

(30) Priority: 09.10.2020 JP 2020171181
(43) Date of publication of application: 19.07.2023
(73) Proprietor: Kabushiki Kaisha Top, Adachi-ku, Tokyo 120-0035 (JP)
(72) Inventor: IKEUCHI, Atsushi, Tokyo 120-0035 (JP)
(74) Representative: Schön, Christoph
(86) International application number: PCT/JP2021/036610
(87) International publication number: WO 2022/075255

(56) References cited:
- US-A1- 2017 049 513
- US-A1- 2018 193 652
- US-A1- 2020 179 697

## Description

### Technical Field

The present invention relates to a high-frequency treatment device for performing a treatment such as thermal coagulation of nerve tissue or ablation of tumor tissue by applying a high-frequency current to a treatment target such as a human or an animal.

### Background Art

In the related art, in the medical field, a high-frequency treatment in which an electrode is disposed in a body of a human or an animal and a high-frequency current is passed from the electrode to perform ablation of a tissue is widely used. Further, in recent years, as one of pain treatments, a method of performing nerve blocking by a high-frequency treatment has attracted attention (for example, refer to Patent Literature 1). The nerve blocking by the high-frequency treatment has advantages in that there are few side effects on surrounding tissues and the effect lasts for a long period, as compared with the method using a drug in the related art.

There are two types of nerve blocking by this high-frequency treatment, these methods are a high-frequency thermal coagulation method and a pulse high-frequency method, and the high-frequency thermal coagulation method is a method of heating a part of a nerve tissue at a temperature of approximately 80°C for several minutes with a high-frequency current flowing from electrodes and thermally coagulating the nerve tissue, thereby blocking pain signals. In addition, the pulse high-frequency method is a method in which a high-frequency current is intermittently passed through a part of a nerve tissue to heat the nerve tissue at a temperature of 42°C or lower for a dozen minutes, thereby blocking pain signals without damaging the nerve.
US 2020/179697 A1 discloses a system and method for selectively and reversibly modulating targeted neural and non-neural tissue of a nervous system for the treatment of pain, wherein an electrical stimulation is delivered to the treatment site that selectively and reversibly modulates the targeted neural- and non-neural tissue of the nervous structure, inhibiting pain while preserving other sensory and motor function, and proprioception. US 2017/049513 A1 discloses a system and a method for applying radiofrequency electrical energy to a living body comprising at least three electrodes, each of the at least three electrodes having an exposed conductive tip and a temperature sensor in the conductive tip, each electrode being adapted to be inserted into a patient's body so that said conductive tip will contact the tissue in the patient's body and the temperature sensor will sense the temperature of the tissue near said conductive tip, a generator that produces high-frequency signal output between at least two output poles, a temperature detector that measures the temperatures from the temperature sensors, a switching system by which each of the at least three electrodes can be connected to and disconnected from each of the at least two output poles, and a controller that automatically produces a sequence of steps to regulate at the same time the temperatures measured from all of the at least three electrodes, such that the temperature measured from each of the at least three electrodes is held at a set temperature for the electrode; wherein, for each of the steps of the sequence, the controller configures the switching system to connect one or more of the at least three electrodes to a first output pole of the least two output poles forming a first group of electrodes, and to connect one or more of the at least three electrodes to a second output pole of the at least two output poles forming a second group of electrodes, so that high-frequency signal output is electrically conducted through the patient's body between the first group of electrodes and the second group of electrodes.

### Citation List

### Patent Literature

Patent Literature 1: PCT Japanese Translation Patent Publication No. 2018-511444

### Summary of Invention

### Technical Problem

In the high-frequency thermal coagulation method, the nerve tissue is heated at a high temperature of approximately 80°C, and thus a patient as a treatment target feels pain due to the heating of the nerve tissue. Therefore, before a start of the high-frequency treatment, an anesthetic is injected into a portion at which the high-frequency treatment is to be performed, and local anesthesia is performed so that the pain caused by heating is not felt. Meanwhile, depending on a constitution of the patient and the portion to which the high-frequency treatment is to be performed, a temperature of the nerve tissue is increased before the anesthesia is effective, and the patient feels pain during the high-frequency treatment, in some cases.

In view of such circumstances, the present invention has an object to provide a high-frequency treatment device capable of reducing pain felt by a patient as a treatment target during a treatment.

### Solution to Problem

According to an aspect of the present invention, there is provided a high-frequency treatment device including: an output unit configured to output high-frequency power; a plurality of electrodes connected to the output unit and configured to be disposed in or on a patient; a temperature measurement unit configured to measure an ambient temperature of at least one of the electrodes; a control unit configured to perform normal output control of controlling the output of the output unit so that a temperature measurement value measured by the temperature measurement unit is equal to a control target value; and an operation detection unit configured to detect an operation of the patient, characterized in that the control unit is configured to perform output reduction control of reducing the output of the output unit below the output of the output unit in the normal output control, based on the operation detection unit detecting the operation of the patient.

With the present invention, in a case where a patient as the treatment target feels pain due to heating, it is possible to reduce the output of the high-frequency power by the patient's own operation, and thus it is possible to reduce the pain felt by the patient during the treatment.

In addition, in the aspect of the present invention, the control unit may perform the output reduction control only while the operation detection unit detects the operation of the treatment target.

According to this, when the patient as the treatment target cancels the operation, it is possible to return the output reduction control to the normal output control. Accordingly, it is possible for the patient to determine a timing at which the output of the high-frequency power is returned to the normal output, and thus it is possible to more effectively reduce the pain felt by the patient.

Further, in the aspect of the present invention, the control unit may perform the output reduction control for a predetermined period, based on the operation detection unit detecting the operation of the treatment target.

According to this, even when it is difficult to continue the operation in a case where the patient as the treatment target feels pain, it is possible to perform the output reduction control only for a predetermined period, and it is possible to more effectively reduce the pain felt by the patient.

Further, in the aspect of the present invention, in a case where the temperature measurement value is equal to or lower than a preset temperature reference value, the control unit may perform the normal output control even when the operation detection unit detects the operation of the treatment target.

According to this, it is possible to prevent the ambient temperature of the electrode from being significantly lowered while controlling the output reduction by the pain felt by the patient as the treatment target. Accordingly, it is possible to shorten a time required for the ambient temperature of the electrode to be increased to a temperature required for the treatment after returning to the normal output control, and thus it is possible to perform the treatment more efficiently.

Further, in the aspect of the present invention, the control unit may set, in the normal output control, the control target value to a first target value and control the output of the output unit so that the temperature measurement value is equal to the control target value, and set, in the output reduction control, the control target value to a second target value lower than the first target value and control the output of the output unit so that the temperature measurement value is equal to the control target value.

According to this, it is possible to perform the normal output control and the output reduction control with one control algorithm, and thus it is possible to prevent the control algorithm from becoming complicated. Further, it is possible to prevent occurrence of a bug caused by a complicated control algorithm and a cost increase in apparatus configuration.

In addition, in the aspect of the present invention, in the output reduction control, the control unit may set the control target value to a temperature lower than the current temperature measurement value.

According to this, it is possible to reliably reduce the output of the high-frequency power regardless of the temperature at which the operation of the patient as the treatment target is detected, and thus it is possible to reliably reduce the pain felt by the patient.

In addition, in the aspect of the present invention, the operation detection unit may be configured to change a first voltage value and a second voltage value to be input to the control unit in a case where the operation of the treatment target is received, and the control unit may determine whether or not the operation detection unit detects the operation of the treatment target based on a change of the first voltage value and the second voltage value.

According to this, by comparing the first voltage value and the second voltage value, it becomes possible to detect a failure of the operation detection unit, thereby improving safety and preventing a defect due to an erroneous detection of the operation detection unit.

According to a high-frequency treatment device according to the present invention, it is possible to achieve an excellent effect in that pain felt by a patient as a treatment target during a treatment is reduced.

### Brief Description of Drawings

FIG. 1 is a schematic view illustrating an external appearance of a high-frequency treatment device according to a first embodiment of the present invention.
FIG. 2 is a block diagram illustrating an internal configuration of the high-frequency treatment device according to the first embodiment.
FIGS. 3A and 3B are schematic views illustrating a configuration of an operation switch.
FIGS. 4A and 4B are schematic views illustrating an operation state in a quadpolar manner.
FIG. 5 is a flowchart illustrating an outline of an output control flow.
FIG. 6 is a time chart illustrating an example of a change in control state and temperature measurement value of a main control unit in a high-frequency treatment.
FIGS. 7A and 7B are schematic views illustrating a modification example of the operation switch.
FIG. 8 is a block diagram illustrating an internal configuration of a high-frequency treatment device according to a second embodiment.
FIGS. 9A to 9C are schematic views illustrating an operation state in a tripolar manner.

### Description of Embodiments

Hereinafter, embodiments of the present invention will be described with reference to the accompanying drawings.

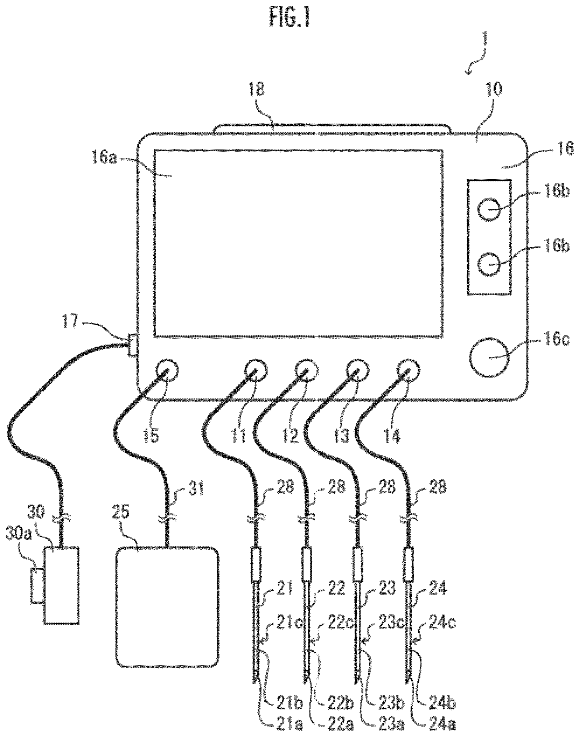

FIG. 1 is a schematic view illustrating an external appearance of a high-frequency treatment device 1 according to a first embodiment of the present invention. The high-frequency treatment device 1 of the present embodiment is for performing nerve blocking by partially heating a peripheral nerve with a high-frequency current. As illustrated in FIG. 1, the high-frequency treatment device 1 includes a main body 10, four electrodes 21, 22, 23, and 24 connected to the main body 10, a counter electrode plate 25 connected to the main body 10, and an operation switch 30 connected to the main body 10.

The main body 10 accommodates or supports an internal configuration, which will be described below. On a front surface of the main body 10, four electrode connectors 11 to 14 to which the electrodes 21 to 24 are respectively connected and a counter electrode plate connector 15 to which the counter electrode plate 25 is connected are provided at a lower portion. An operation unit 16 that accepts an operation of a user is further provided on the front surface of the main body 10. The operation unit 16 is configured with a touch panel display 16a that accepts input operations such as various settings and displays various types of information, a button 16b that receives start and end operations of a treatment, and a control knob 16c for adjusting an output power.

An operation switch connector 17 to which the operation switch 30 is connected is provided on a left side surface of the main body 10. Further, a handle 18 for carrying the main body 10 is provided on an upper portion of the main body 10. Although not illustrated, a rear surface of the main body 10 is provided with a power connector and a power switch which are connected to a commercial alternating current power supply to receive a supply of power.

The electrodes 21 to 24 are inserted into a body of a patient as a treatment target on which a high-frequency treatment is to be performed, and are used to apply a high-frequency current into the body. In the present embodiment, the electrodes 21 to 24 are configured in a needle tubular shape capable of piercing a human body and injecting a drug via the electrodes 21 to 24. The electrodes 21 to 24 have insulating portions 21b to 24b mostly coated with insulation except for non-insulating portions 21a to 24a at tip portions, and the high-frequency current is output from the non-insulating portions 21a to 24a.

Further, thermocouples 21c to 24c for measuring a treatment temperature are incorporated in the electrodes 21 to 24. The electrodes 21 to 24 are electrically connected to the main body 10 via electrode cables 28 and the electrode connectors 11 to 14. The electrodes 21 to 24 may have other shapes, and may be rod-shaped, for example, inserted into a needle tube or a catheter. Further, the thermocouples 21c to 24c may be provided separately from the electrodes 21 to 24.

The counter electrode plate 25 is a flat plate-shaped electrode that is disposed to be attached on a skin surface of the patient as a treatment target, and is for passing the high-frequency current between the electrodes 21 to 24 inserted into the inside. That is, the counter electrode plate 25 is used in a case where the high-frequency current flows in a so-called monopolar manner. The counter electrode plate 25 is electrically connected to the main body 10 via a counter electrode plate cable 31 and the counter electrode plate connector 15. In the present embodiment, the counter electrode plate 25 is configured in a rectangular flat plate shape, and the counter electrode plate 25 may have another shape.

The operation switch 30 is provided to the patient as a treatment target during a treatment, and is operated by the patient. The operation switch 30 includes a push button 30a that is pressed by the patient, and is electrically connected to the main body 10 via the operation switch connector 17.

In the present embodiment, in a case where the patient feels pain during the treatment, the patient presses the push button 30a to discriminate whether or not the patient feels the pain. Then, an output of the high-frequency power is reduced based on the fact that the operation switch 30 detects the operation of the patient, thereby reducing the pain of the patient. That is, an operation detection unit of the present invention is configured with the operation switch 30.

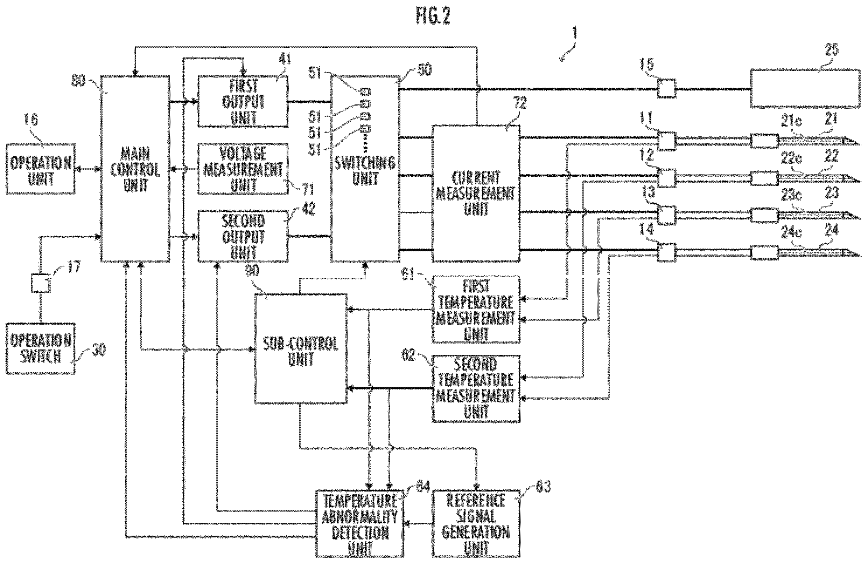

FIG. 2 is a block diagram illustrating an internal configuration of the high-frequency treatment device 1. As illustrated in FIG. 2, the high-frequency treatment device 1 includes, as the internal configuration, a first output unit 41 and a second output unit 42, a switching unit 50, a first temperature measurement unit 61 and a second temperature measurement unit 62, a reference signal generation unit 63, a temperature abnormality detection unit 64, a voltage measurement unit 71, a current measurement unit 72, a main control unit 80, and a sub-control unit 90.

Based on power supplied from the commercial alternating current power supply, the first output unit 41 and the second output unit 42 generate and output high-frequency power with a preset frequency (for example, 470 to 490 kHz) and a voltage based on an output control signal from the main control unit 80 (for example, 18 to 22 Vrms). Each of the first output unit 41 and the second output unit 42 is configured with a known circuit having a transformer, whereby the body as a treatment target is insulated from the commercial alternating current power supply.

The first output unit 41 is connected to the electrode connector 11, the electrode connector 12, and the electrode connector 13 and the counter electrode plate connector 15 via the switching unit 50, that is, is connected to be capable of outputting the high-frequency power to the electrode 21, the electrode 22, and the electrode 23 and the counter electrode plate 25. Further, the second output unit 42 is connected to the electrode connector 13, the electrode connector 14, and the counter electrode plate connector 15 via the switching unit 50, that is, is connected to be capable of outputting the high-frequency power to the electrode 23, the electrode 24, and the counter electrode plate 25.

Therefore, an output unit of the present invention is configured with the first output unit 41 and the second output unit 42. In the present embodiment, by providing two output units of the first output unit 41 and the second output unit 42, it is possible to pass the high-frequency current while simultaneously performing output control in a stable manner with two electrode sets (for example, an electrode set of the electrode 21 and the electrode 22 and an electrode set of the electrode 23 and the electrode 24).

The switching unit 50 operates under control of the sub-control unit 90, and switches the connection between the first output unit 41 and the second output unit 42, and the electrode connectors 11 to 14 and the counter electrode plate connector 15. That is, the switching unit 50 switches to which electrode set of electrode sets, in which at least two of the electrodes 21 to 24 and the counter electrode plate 25 are combined, the high-frequency current flows.

The switching unit 50 is configured with a circuit including a plurality of switches 51 including a semiconductor switch such as a metal oxide semiconductor field effect transistor (MOSFET) or a reed relay, and is provided between the first output unit 41 and the second output unit 42, and the electrodes 21 to 24 and the counter electrode plates 25.

In the present embodiment, since the two output units of the first output unit 41 and the second output unit 42 are provided, by reducing the number of switches 51 and simplifying the configuration of the switching unit 50, it is possible to speed up switching of connection between the first output unit 41 and the second output unit 42, and each of the electrodes 21 to 24.

The first temperature measurement unit 61 and the second temperature measurement unit 62 are configured with a known circuit, generate a temperature measurement signal (for example, 25 mV/°C) based on signals received from the thermocouples 21c to 24c, and transmit the temperature measurement signal to the sub-control unit 90 and the temperature abnormality detection unit 64. The first temperature measurement unit 61 is connected to the thermocouple 21c incorporated in the electrode 21 and the thermocouple 23c incorporated in the electrode 23. In addition, the second temperature measurement unit 62 is connected to the thermocouple 22c incorporated in the electrode 22 and the thermocouple 24c incorporated in the electrode 24. That is, a temperature measurement unit of the present invention is configured with the thermocouples 21c to 24c, the first temperature measurement unit 61 and the second temperature measurement unit 62.

In the present embodiment, by providing two temperature measurement units of the first temperature measurement unit 61 and the second temperature measurement unit 62, it is possible to accurately measure ambient temperatures of all the four electrodes 21 to 24 in a short time. That is, while ensuring a sufficient measurement time for each of the thermocouples 21c to 24c, the entire measurement time is not lengthened.

In addition, the first temperature measurement unit 61 and the second temperature measurement unit 62 respectively measure each of both ambient temperatures of the electrodes 21, 22, or 23 from which a high-frequency power is output from the first output unit 41 and ambient temperatures of the electrodes 23 and 24 from which a high-frequency power is output from the second output unit 42, so that even in a case where either the first temperature measurement unit 61 or the second temperature measurement unit 62 fails, occurrence of a temperature abnormality by the first output unit 41 or the second output unit 42 is detectable, thereby improving safety.

The reference signal generation unit 63 is configured with a known circuit, and generates a temperature reference signal serving as a reference for determining whether or not the temperature measured by the first temperature measurement unit 61 or the second temperature measurement unit 62 is an abnormal temperature. The reference signal generation unit 63 is controlled by the sub-control unit 90 to generate, for example, the temperature reference signal corresponding to a temperature setting value of +7°C. Here, the temperature setting value is input to the operation unit 16 as a temperature maintained during the treatment, acquired by the main control unit 80, and transmitted to the sub-control unit 90. The generated temperature reference signal is transmitted to the temperature abnormality detection unit 64.

The temperature abnormality detection unit 64 is configured with a known circuit, and compares a temperature reference signal received from the reference signal generation unit 63 with a temperature measurement signal received from the first temperature measurement unit 61 or the second temperature measurement unit 62 and detects the occurrence of the temperature abnormality. In a case where a voltage of the temperature measurement signal is higher than a voltage of the temperature reference signal, the temperature abnormality detection unit 64 transmits an output stop signal to the first output unit 41, the second output unit 42, and the main control unit 80. Upon receiving the output stop signal, the first output unit 41 and the second output unit 42 stop the output of the high-frequency power. In addition, the main control unit 80 that receives the output stop signal performs a temperature abnormality process such as an alarm display.

The voltage measurement unit 71 is configured with a known circuit, and individually measures a voltage of the high-frequency power output by the first output unit 41 and the second output unit 42. The voltage measurement unit 71 generates a voltage measurement signal based on a high-frequency voltage generated by the first output unit 41 and the second output unit 42, and transmits the voltage measurement signal to the main control unit 80.

The current measurement unit 72 is configured with a known circuit, and individually measures a high-frequency current flowing through the electrodes 21 to 24. The current measurement unit 72 generates a current measurement signal based on the high-frequency current flowing through the electrodes 21 to 24, and transmits the current measurement signal to the main control unit 80.

The main control unit 80 includes a known configuration of a CPU, a ROM, and a RAM, and controls each unit of the high-frequency treatment device 1 such as the operation unit 16, the first output unit 41 and the second output unit 42, to execute the high-frequency treatment. Based on the input operation accepted by the operation unit 16, the main control unit 80 transmits a connection mode signal indicating a connection mode of the first output unit 41 and the second output unit 42, and the electrode connectors 11 to 14 and the counter electrode plate connector 15 to the sub-control unit 90.

The main control unit 80 also starts or ends the output of the high-frequency power from the first output unit 41 or the second output unit 42 based on the input operation accepted by the operation unit 16, and also performs the output control of the high-frequency power with PID control so that a temperature measurement value received from the sub-control unit 90 is substantially equal to a control target value. That is, a control unit of the present invention is configured with the main control unit 80.

As the output control, the main control unit 80 performs two types of control of normal output control and output reduction control in which the output of the high-frequency power is reduced than the normal output control. In the present embodiment, in the normal output control, the main control unit 80 sets the control target value to the temperature setting value (first target value) input to the operation unit 16, and performs the output control. Further, in the output reduction control, the main control unit 80 performs the control by setting the control target value to a second target value, which is a value lower than the current temperature measurement value by 10°C.

The output reduction control is performed to reduce pain felt by the patient as a treatment target. Therefore, in a case where the operation switch 30 detects an operation of the patient, the main control unit 80 shifts the output control from the normal output control up to that point to the output reduction control. Details of the output control will be described below.

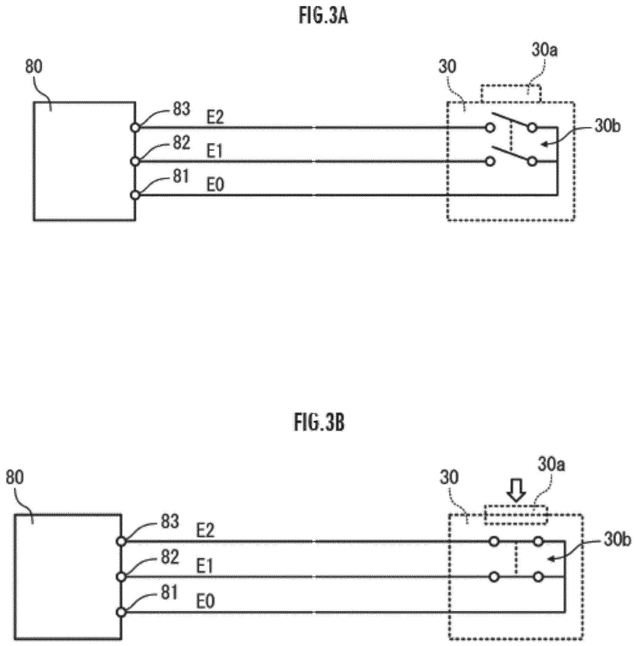

FIGS. 3A and 3B are schematic views illustrating a configuration of the operation switch 30. As illustrated in FIGS. 3A and 3B, the operation switch 30 includes a bipolar single-throw switch 30b that is operated by operating the push button 30a. The bipolar single-throw switch 30b is disposed so as to switch between connection and disconnection of the output terminal 81, and the first input terminal 82 and the second input terminal 83 of the main control unit 80.

Specifically, as illustrated in FIG. 3A, in a case where the patient as a treatment target does not press the push button 30a, the bipolar single-throw switch 30b maintains an off-state, and the output terminal 81, and the first input terminal 82 and the second input terminal 83 are disconnected. Then, as illustrated in FIG. 3B, in a case where the patient presses the push button 30a, the bipolar single-throw switch 30b is turned on, and the output terminal 81 is connected to the first input terminal 82 and the second input terminal 83.

When the output terminal 81 is connected to the first input terminal 82 and the second input terminal 83, a voltage applied to the output terminal 81 is also applied to the first input terminal 82 and the second input terminal 83. Specifically, in a state in which the patient does not press the push button 30a, both a first voltage value E1 input to the first input terminal 82 and a second voltage value E2 input to the second input terminal 83 become 0 V (reference potential). In a state in which the patient is pressing the push button 30a, the first voltage value E1 and the second voltage value E2 are equal to a detection voltage value E0 (for example, 3.3 V) output from the output terminal 81. In other words, the operation switch 30 is configured to change the first voltage value E1 and the second voltage value E2 that are input to the main control unit 80 in a case where the operation switch 30 receives the operation of the patient.

The main control unit 80 monitors the voltages input to the first input terminal 82 and the second input terminal 83, and determined whether or not the operation switch 30 detects the operation of the patient as a treatment target based on the change in the first voltage value E1 and the second voltage value E2. Specifically, the main control unit 80 determines that the operation switch 30 does not detect the operation of the patient in a case where both the first voltage value E1 and the second voltage value E2 are 0 V. The main control unit 80 also determines that the operation switch 30 detects the operation of the patient in a case where both the first voltage value E1 and the second voltage value E2 are the detection voltage value E0.

In addition, in a case where the first voltage value E1 and the second voltage value E2 are different values from each other, the main control unit 80 determines that a failure occurs in any portion, and causes the touch panel display 16a to display an alarm. In this manner, the operation switch 30 is configured to change the first voltage value E1 and the second voltage value E2 input to the main control unit 80 in a case of receiving the operation of the patient, so that it is possible to improve safety and prevent a defect caused by an erroneous detection of the operation switch 30. That is, it is possible to prevent defects such as the output not being reduced even though the patient as a treatment target feels pain, or the temperature measurement value not rising to the temperature setting value even though the patient does not feel pain.

Further, the main control unit 80 calculates a voltage measurement value, a current measurement value, a power measurement value, and an impedance measurement value, based on the voltage measurement signal received from the voltage measurement unit 71 and the current measurement signal received from the current measurement unit 72, and displays the voltage measurement value, the current measurement value, the power measurement value, and the impedance measurement value on the touch panel display 16a together with the temperature measurement value.

The main control unit 80 uses an output voltage as an operation amount in the high-frequency thermal coagulation method and an output pulse width as an operation amount in the pulse high-frequency method. Further, in a case where an output voltage value is set by an input operation accepted by the operation unit 16, the main control unit 80 performs the output control of the high-frequency power with PID control so that the voltage measurement value becomes equal to a voltage setting value.

In the same manner as the main control unit 80, the sub-control unit 90 has a known configuration of a CPU, a ROM, and a RAM, and controls the switching unit 50, the first temperature measurement unit 61, the second temperature measurement unit 62, and the reference signal generation unit 63. The sub-control unit 90 controls ON and OFF of the plurality of switches 51 included in the switching unit 50 based on a connection mode signal transmitted from the main control unit 80, and switches connection states of the first output unit 41 and the second output unit 42, the electrode connectors 11 to 14, and the counter electrode plate connector 15.

The sub-control unit 90 also calculates a temperature measurement value based on the temperature measurement signal received from the first temperature measurement unit 61 or the second temperature measurement unit 62, and transmits the temperature measurement value to the main control unit 80. Further, the sub-control unit 90 controls the reference signal generation unit 63 based on the temperature setting value received from the main control unit 80 to generate a temperature reference signal, and transmits the temperature reference signal to the temperature abnormality detection unit 64.

The switching unit 50, the first temperature measurement unit 61, the second temperature measurement unit 62, the reference signal generation unit 63, the temperature abnormality detection unit 64, the voltage measurement unit 71, the current measurement unit 72, and the sub-control unit 90 are insulated from the commercial alternating current power supply, in order to protect the body as a treatment target from a high-voltage current.

Next, an operation of the high-frequency treatment device 1 will be described.

The high-frequency treatment device 1 is capable of four types of output formats of a high-frequency current to a treatment target: monopolar, bipolar, tripolar, and quadpolar. In the present embodiment, the monopolar manner has an output format in which a high-frequency current flows through an electrode set in which any one of the electrodes 21 to 24 and the counter electrode plate 25 are combined, and the bipolar manner has an output format in which a high-frequency current flows by using the electrodes 21 and 22 or the electrodes 23 and the electrode 24 as an electrode set.

In addition, the tripolar manner has an output format in which a high-frequency current flows between the electrode 21, and the electrode 22 and the electrode 23, between the electrode 22, and the electrode 21 and the electrode 23, or between the electrode 23, and the electrode 21 and the electrode 22, by using the electrode 21, the electrode 22, and the electrode 23 as an electrode set. Further, in the tripolar manner, it is also possible to switch a state in which a high-frequency current flows between the electrode 21, and the electrode 22 and the electrode 23, a state in which a high-frequency current flows between the electrode 22, and the electrode 21 and the electrode 23, and a state in which a high-frequency current flows between the electrode 23, and the electrode 21 and the electrode 22, at a specific cycle (for example, 0.1 seconds).

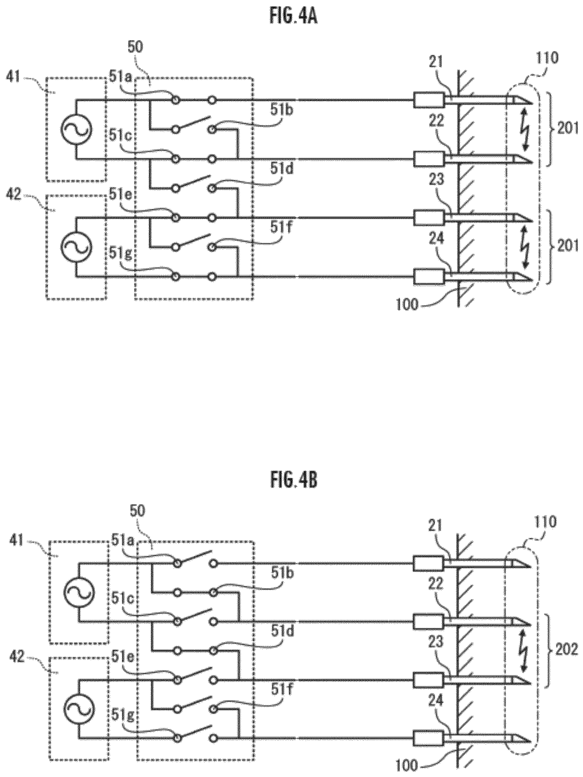

Hereinafter, the operation of the high-frequency treatment device 1 will be described by using a case where an output format is quadpolar, as an example. FIGS. 4A and 4B are schematic views illustrating an operation state in a quadpolar manner. In order to facilitate understanding, FIGS. 4A and 4B describe only switches 51a to 51g necessary for description among the plurality of switches 51 included in the switching unit 50.

As illustrated in FIGS. 4A and 4B, in a case where a high-frequency treatment in a quadpolar manner is performed, first, all the four electrodes 21 to 24 are inserted into a treatment target 100. At this time, the electrodes 21 to 24 are arranged in a row at substantially equal intervals, for example, in this order.

When the electrodes 21 to 24 are arranged, nerve exploration, measurement of an impedance of the peripheral tissue of the electrodes 21 to 24, are performed. Meanwhile, since the nerve exploration and the measurement are techniques in the related art, the description thereof will be omitted. In addition, before or after the insertion of the electrodes 21 to 24, an anesthetic is injected in the vicinity of disposition positions of the electrodes 21 to 24, and local anesthesia is performed to reduce pain due to overheating during the treatment.

When the electrodes 21 to 24 are appropriately arranged and the operation unit 16 accepts a quadpolar manner selection operation by a user such as a doctor, the main control unit 80 transmits a connection mode signal indicating a quadpolar connection mode to the sub-control unit 90. When receiving the connection mode signal indicating the quadpolar connection mode from the main control unit 80, the sub-control unit 90 controls the switching unit 50, and sets a connection state of the first output unit 41 and the second output unit 42, and the electrodes 21 to 24 to be a first state illustrated in FIG. 4A.

Specifically, in the first state, the sub-control unit 90 turns on the switch 51a, the switch 51c, the switch 51e, and the switch 51g, and turns off the switch 51b, the switch 51d, and the switch 51f. Accordingly, the first output unit 41 is connected to the electrode 21 and the electrode 22, and the second output unit 42 is connected to the electrode 23 and the electrode 24. Therefore, a state in which a high-frequency current by high-frequency power output by the first output unit 41 flows between the electrodes 21 and 22, and a high-frequency current by the high-frequency power output by the second output unit 42 flows between the electrodes 23 and 24 is obtained. As a result, a first electrode set 201, which is an electrode set through which a high-frequency current flows in the first state, is configured with the electrodes 21 and 22, and the electrodes 23 and 24, respectively.

After that, when the operation unit 16 accepts a start operation of a treatment after the operation unit 16 receives an operation of selecting the high-frequency thermal coagulation method or the pulse high-frequency method and an input operation of a temperature setting value (for example, 80°C) and a treatment time (for example, 3 minutes) by the user (in a case where the pulse high-frequency method is selected, the main control unit 80 sets the temperature setting value to 42°C, so that only the treatment time is input), the main control unit 80 controls the first output unit 41 and the second output unit 42 to start an output of high-frequency power. In addition, the main control unit 80 transmits an output start signal indicating that the output is started to the sub-control unit 90.

When the output start signal is received from the main control unit 80, the sub-control unit 90 controls the switching unit 50 so that the connection state of the first output unit 41 and the second output unit 42, and the electrodes 21 to 24 is switched between the first state illustrated in FIG. 4A in a specific cycle (in the present embodiment, 0.1 seconds) and a second state illustrated in FIG. 4B.

In the second state, the sub-control unit 90 turns on the switch 51b and the switch 51d, and turns off the switch 51a, the switch 51c, the switch 51e, the switch 51f, and the switch 51g, as illustrated in FIG. 4B. Accordingly, a state is obtained in which the first output unit 41 is connected to the electrode 22 and the electrode 23, and a high-frequency current by the high-frequency power output by the first output unit 41 flows between the electrode 22 and the electrode 23. As a result, a second electrode set 202, which is an electrode set through which a high-frequency current flows in the second state, is configured with the electrode 22 and the electrode 23.

During the output of the high-frequency power, with switching control of the switching unit 50 by the sub-control unit 90, the first state in which a high-frequency current flows between the electrodes 21 and 22 and between the electrodes 23 and 24 and the second state in which a high-frequency current flows between the electrodes 22 and 23 are periodically switched. Accordingly, each of a region between the electrodes 21 and 22 in the periphery of the electrodes 21 and 22, a region between the electrodes 22 and 23 in the periphery of the electrodes 22 and 23, and a region between the electrodes 23 and 24 in the periphery of the electrodes 23 and 24 is heated intermittently. Meanwhile, in the present embodiment, the switching cycle is set to 0.1 seconds, so that a temperature of each region does not drop too much when not being heated.

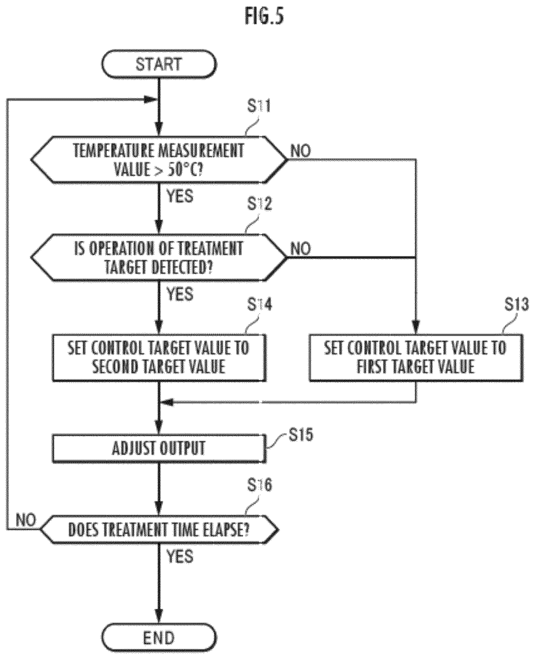

The main control unit 80 performs the output control of the first output unit 41 and the second output unit 42 in parallel with the switching control of the switching unit 50 by the sub-control unit 90. FIG. 5 is a flowchart illustrating an outline of a flow of output control.

In the output control, first, in step S11, the main control unit 80 determines whether or not a temperature measurement value received from the sub-control unit 90 is larger than 50°C which is a temperature reference value. In a case where the received temperature measurement value is larger than 50°C, the process proceeds to step S12, and in a case where the received temperature measurement value is equal to or less than 50°C, the process proceeds to step S14.

In step S12, the main control unit 80 determines whether or not the operation switch 30 detects an operation of a patient as a treatment target, based on the first voltage value E1 and the second voltage value E2. In a case where the operation switch 30 detects the operation of the treatment target, the process proceeds to step S13, and in a case where the operation switch 30 does not detect the operation of the treatment target, the process proceeds to step S14.

In step S13, the main control unit 80 sets a control target value to a first target value, that is, a temperature setting value input to the operation unit 16. Accordingly, the main control unit 80 normally performs output control. In step S13, the main control unit 80 also transmits the set control target value to the sub-control unit 90.

In step S14, the main control unit 80 sets the control target value to a second target value, that is, a value lower than a current temperature measurement value referred to in step S12 by 10°C. Accordingly, the main control unit 80 performs output reduction control. In step S14, the main control unit 80 also transmits the set control target value to the sub-control unit 90.

In step S15, the main control unit 80 controls an output of high-frequency power from the first output unit 41 and the second output unit 42 with PID control. Specifically, output voltages from the first output unit 41 and the second output unit 42 are adjusted so that the temperature measurement value received from the sub-control unit 90 is substantially equal to the control target value set in step S13 or S14.

In step S16, it is determined whether or not a treatment time input to the operation unit 16 elapses. In a case where the treatment time elapses, the output control is ended, and in a case where the treatment time does not elapse, the process returns to step S11, and the processes of steps S11 to S16 are repeated.

When the output control is ended, the main control unit 80 stops the output of the high-frequency power from the first output unit 41 and the second output unit 42. The main control unit 80 also transmits an output end signal to the sub-control unit 90. The sub-control unit 90 that receives the output end signal from the main control unit 80 stops switching control between the first state and the second state with respect to the switching unit 50. With the above, one treatment is completed.

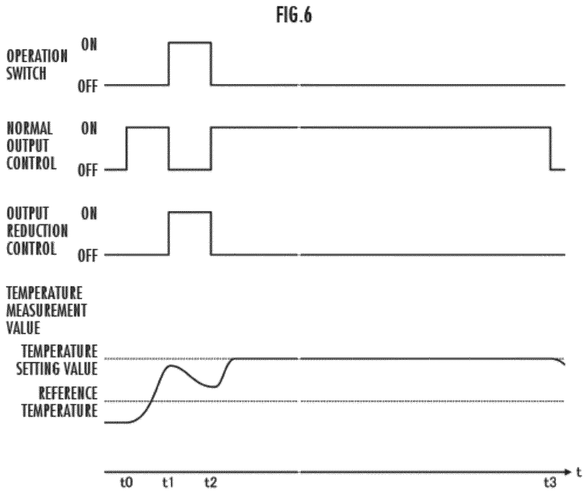

FIG. 6 is a time chart illustrating an example of a change in control state and temperature measurement value of the main control unit 80 in a high-frequency treatment. In FIG. 6, a horizontal axis is a time t. In addition, the change in temperature measurement value illustrated in FIG. 6 is not always accurate.

As illustrated in FIG. 6, in the high-frequency treatment, the main control unit 80 first starts the first output unit 41 and the second output unit 42 to output high-frequency power, and also starts normal output control, at a time t0. Accordingly, the temperature measurement value, that is, an ambient temperature of the electrodes 21 to 24 is increased toward a temperature setting value (first target value).

When a patient as a treatment target feels pain at a time t1 when the temperature measurement value exceeds a temperature reference value and approaches the temperature setting value, and the operation switch 30 is operated, the main control unit 80 changes the normal output control to output reduction control. In the output reduction control, a temperature lower than the current temperature measurement value by 10°C is set as a control target value, so that the temperature measurement value is decreased. Further, during the output reduction control, the control target value becomes a low value each time the process of step S14 is repeated, and the temperature measurement value does not become equal to the control target value. Therefore, the temperature measurement value is continuously decreased.

When the patient does not feel pain by the decrease in the ambient temperature of the electrodes 21 to 24 and the effect of the prior local anesthesia and the operation of the operation switch 30 is released at a time t2, the main control unit 80 changes the output reduction control to the normal output control. Accordingly, the temperature measurement value is increased toward the temperature setting value again, and after reaching the temperature setting value, the temperature measurement value is maintained at a temperature substantially equal to the temperature setting value. Accordingly, thermal coagulation or degeneration of tissues occurs.

After that, at a time t3 when a treatment time elapses, the main control unit 80 ends the normal output control, and ends the output of the high-frequency power from the first output unit 41 and the second output unit 42. A nerve tissue contained in a region 110 is heated at a temperature of, for example, 80°C for approximately 3 minutes, and as a result, a pain signal is blocked.

In this manner, with the high-frequency treatment device 1, in a case where the patient as a treatment target feels pain due to heating, it is possible to shift the output control to the output reduction control by the patient's own operation, and thus, it is possible to reduce the pain felt by the patient during the treatment. Further, in the present embodiment, since the output reduction control is performed only during the operation of the operation switch 30, it is also possible for the patient to determine a timing for returning to the normal output control, and it is possible to more effectively reduce the pain felt by the patient.

In addition, in the present embodiment, since the output reduction control is realized by changing the control target value, it is possible to prevent complication of a control algorithm and occurrence of bugs associated with the control algorithm, and a cost increase in apparatus configuration. Further, in the present embodiment, since the control target value in the output reduction control is set to a temperature lower than the current temperature measurement value by 10°C, it is possible to reliably reduce the output, regardless of a temperature when the operation switch 30 is operated.

Further, in the present embodiment, since the output reduction control is not performed in a case where the temperature measurement value is lower than the temperature reference value, it is possible to prevent the ambient temperature of the electrodes 21 to 24 from dropping too much even during the output reduction control. Accordingly, it is possible to shorten a time required for the ambient temperature of the electrodes 21 to 24 to rise to the temperature setting value after the output reduction control, and to improve efficiency of the treatment.

In a case where the temperature measurement value drops to the temperature reference value during the output reduction control, the normal output control and the output reduction control are periodically switched, and the temperature measurement value (ambient temperature of the electrodes 21 to 24) is maintained at a temperature substantially equal to the temperature reference value. In addition, in a case where the operation switch 30 is operated in a state in which the temperature measurement value is equal to or lower than the temperature reference value, the normal output control and the output reduction control are periodically performed after the temperature measurement value is increased to the temperature reference value by the normal output control, so that the temperature measurement value (ambient temperature of the electrodes 21 to 24) is maintained at a temperature substantially equal to the temperature reference value.

Further, in the present embodiment, since the temperature reference value is set to 50°C, which is higher than the temperature setting value of 42°C in the pulse high-frequency method, the output reduction control is not performed in the pulse high-frequency method. In other words, in the present embodiment, by setting the temperature reference value in this manner, it is possible to perform the output control at a pulse high-frequency, which does not require for the output reduction control since the temperature setting value is low, with the same control algorithm as the high-frequency thermal coagulation method.

Next, a modification example of the high-frequency treatment device 1 will be described.

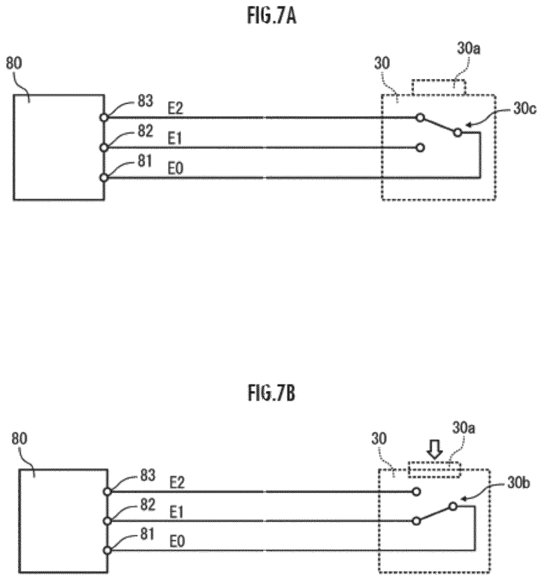

FIGS. 7A and 7B are schematic views illustrating a modification example of the operation switch 30. As illustrated in FIGS. 7A and 7B, the operation switch 30 in this example includes a single-pole double-throw switch 30c that is operated by operating a push button 30a. The single-pole double-throw switch 30c is disposed so as to switch the connection of the output terminal 81 of the main control unit 80 with the first input terminal 82 and the second input terminal 83.

Specifically, as illustrated in FIG. 7A, in a case where a patient as a treatment target does not press the push button 30a, the single-pole double-throw switch 30c disconnects the output terminal 81 and the first input terminal 82, and the output terminal 81 and the second input terminal 83 are maintained in a connected state. Then, as illustrated in FIG. 7B, in a case where the patient presses the push button 30a, the single-pole double-throw switch 30c connects the output terminal 81 and the first input terminal 82, and disconnects the output terminal 81 and the second input terminal 83.

Therefore, in a state in which the patient does not press the push button 30a, the first voltage value E1 input to the first input terminal 82 becomes 0 V (reference potential), and the second voltage value E2 input to the second input terminal 83 becomes the detection voltage value E0 (for example, 3.3 V). Further, in a state in which the patient presses the push button 30a, the first voltage value E1 becomes the detection voltage value E0, and the second voltage value E2 becomes 0 V.

Then, the main control unit 80 monitors the voltages input to the first input terminal 82 and the second input terminal 83, and in a case where the first voltage value E1 is 0 V and the second voltage value E2 is the detection voltage value E0, the operation switch 30 determines that the operation of the patient does not detect. In addition, in a case where the first voltage value E1 is the detection voltage value E0 and the second voltage value E2 is 0 V, the main control unit 80 determines that the operation switch 30 detects the operation of the patient. In a case where the first voltage value E1 and the second voltage value E2 are substantially equal to each other, the main control unit 80 determines that a failure occurs in any portion, and causes the touch panel display 16a to display an alarm.

In this manner, the operation switch 30 may be provided with the single-pole double-throw switch 30c, and in this case as well, it is possible to prevent a defect due to an erroneous detection of the operation switch 30.

In addition, although not illustrated, the output reduction control is not performed only while the operation switch 30 detects the operation of the patient as a treatment target, and the output reduction control may be performed for a predetermined period (for example, 10 seconds), based on the fact that the operation switch 30 detects the operation of the patient. When it is difficult to continue to operate the operation switch 30 in a case where the patient feels pain, such control is preferable.

In addition, the second target value may be a value that is another temperature lower than the current temperature measurement value. Further, the second target value may be lower than the temperature setting value (first target value) by a predetermined temperature. In addition, the temperature reference value may be another temperature. Further, without setting of the temperature reference value, the output reduction control may be performed regardless of the temperature measurement value.

Further, instead of changing the control target value in the output reduction control, the output may be reduced by multiplying an operation amount or the temperature measurement value by a predetermined coefficient. In addition, instead of changing the control target value in the output reduction control, during the output reduction control, the output of the high-frequency power from the first output unit 41 and the second output unit 42 may be stopped, or fixed at a predetermined output.

Further, the operation switch 30 may be operated by another portion such as the mouth or the foot, for example. Further, the operation switch 30 may perform wireless communication with the main control unit 80.

Next, a second embodiment of the present invention will be described.

In the same manner as the high-frequency treatment device 1 according to the first embodiment, a high-frequency treatment device 2 according to the second embodiment is for performing nerve blocking by partially heating a peripheral nerve with a high-frequency current. The high-frequency treatment device 2 basically has the same configuration as the high-frequency treatment device 1 except that the high-frequency treatment device 2 includes only three of the electrodes 21, 22 and 23 and a different internal configuration. Therefore, the same reference numerals will be given to the same portions as in the first embodiment, and the description thereof will be omitted, and only the portions different from the first embodiment will be explained, hereinafter.

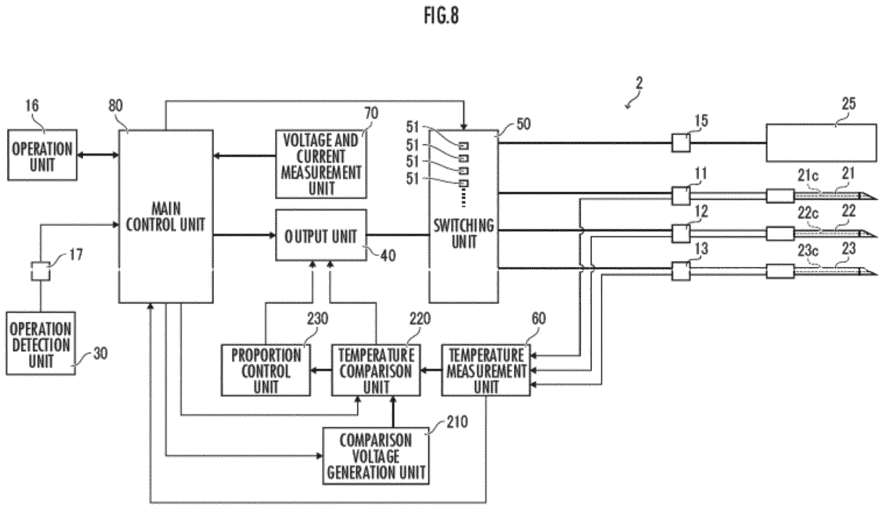

FIG. 8 is a block diagram illustrating the internal configuration of the high-frequency treatment device 2. As illustrated in FIG. 8, the high-frequency treatment device 2 has, as the internal configuration, an output unit 40, the switching unit 50, a temperature measurement unit 60, a comparison voltage generation unit 210, a temperature comparison unit 220, a proportion control unit 230, a voltage and current measurement unit 70, and a main control unit 80.

With a configuration in the same manner as the first output unit 41 and the second output unit 42, based on power supplied from the commercial alternating current power supply, the output unit 40 generates and outputs high-frequency power with a preset frequency (for example, 470 to 490 kHz) and a voltage based on an output control signal from the main control unit 80 (for example, 18 to 22 Vrms). The output unit 40 is connected to the electrode connectors 11 to 13 and the counter electrode plate connector 15 via the switching unit 50, that is, is connected to be capable of outputting the high-frequency power to the electrodes 21 to 23 and the counter electrode plate 25.

In the same manner as the first embodiment, the switching unit 50 is configured with a circuit including the plurality of switches 51 including a semiconductor switch such as a MOSFET or a reed relay, and is provided between the output unit 40, and the electrodes 21 to 23 and the counter electrode plate 25. In the present embodiment, the switching unit 50 operates under control of the main control unit 80, and switches the connection between the output unit 40, and the electrode connectors 11 to 13 and the counter electrode plate connector 15.

The temperature measurement unit 60 is configured with a known circuit, and generates, for example, a temperature measurement signal of 40 mV/°C and transmits the temperature measurement signal to the main control unit 80, and generates, for example, a temperature measurement signal of 20 mV/° C and transmits the temperature measurement signal to the temperature comparison unit 220, based on a signal received from the thermocouples 21c to 23c.

The comparison voltage generation unit 210 is configured with a known circuit, and generates a control reference voltage signal to be used for output control of the output unit 40 and a determination reference voltage signal to be used for determining an abnormality in a measurement temperature. The comparison voltage generation unit 210 is controlled by the main control unit 80 to generate the control reference voltage signal (voltage indicating a control target value) based on the control target value received from the main control unit 80, and transmits the control reference voltage signal to the temperature comparison unit 220. The comparison voltage generation unit 210 also generates the determination reference voltage signal (for example, a voltage indicating a temperature setting value of +7°C) based on the temperature setting value received from the main control unit 80, and transmits the determination reference voltage signal to the temperature comparison unit 220.

The temperature comparison unit 220 is configured with a known circuit, and transmits a difference voltage signal indicating a voltage difference between the control reference voltage signal received from the comparison voltage generation unit 210 and the temperature measurement signal received from the temperature measurement unit 60 to the proportion control unit 230. The temperature comparison unit 220 also compares a voltage of the determination reference voltage signal received from the comparison voltage generation unit 210 with a voltage of the temperature measurement signal received from the temperature measurement unit 60, and transmits an output stop signal to the output unit 40 and the main control unit 80 in a case where the voltage of the temperature measurement signal is higher. The output unit 40 that receives the output stop signal stops the output of the high-frequency power, and the main control unit 80 that receives the output stop signal performs a temperature abnormality process such as an alarm display.

The proportion control unit 230 is configured with a known PWM circuit including a sawtooth wave transmitter and a comparator, and compares the difference voltage signal received from the temperature comparison unit 220 with a sawtooth wave signal to generate a pulse signal, and transmits the pulse signal to the output unit 40. The output unit 40 outputs high-frequency power having a voltage based on a temperature control signal obtained by synthesizing the pulse signal received from the proportion control unit 230 and the output control signal received from the main control unit 80.

The voltage and current measurement unit 70 is configured with a known circuit, and measures a voltage and a current of the high-frequency power output by the output unit 40. The voltage and current measurement unit 70 generates a voltage measurement signal and a current measurement signal based on a high-frequency voltage and a high-frequency current generated by the output unit 40, and transmits the voltage measurement signal and the current measurement signal to the main control unit 80.

The main control unit 80 includes a known configuration of a CPU, a ROM, and a RAM, and controls each unit of the high-frequency treatment device 2 to execute a high-frequency treatment, in the same manner as the first embodiment. The main control unit 80 calculates a temperature measurement value based on the temperature measurement signal received from the temperature measurement unit 60. The main control unit 80 also generates an output control signal based on the calculated temperature measurement value and transmits the output control signal to the output unit 40 to control the output of the high-frequency power together with the proportion control unit 230.

That is, in step S15 of the output control illustrated in FIG. 5, the main control unit 80 of the present embodiment transmits the control target value set in step S13 or S14 to the proportion control unit 230, and generates the output control signal based on the temperature measurement value and transmits the output control signal to the output unit 40.

In addition, the main control unit 80 calculates a voltage measurement value, a current measurement value, a power measurement value, and an impedance measurement value based on the received voltage measurement signal and current measurement signal. Further, in the present embodiment, the main control unit 80 controls the switching unit 50. Specifically, based on an input operation accepted by the operation unit 16, the main control unit 80 controls ON and OFF of the plurality of switches 51 included in the switching unit 50, and switches a connection state of the output unit 40, and the electrode connectors 11 to 13 and the counter electrode plate connector 15.

Next, an operation of the high-frequency treatment device 2 will be described.

The high-frequency treatment device 2 is capable of three types of output formats of a high-frequency current to a treatment target: monopolar, bipolar, and tripolar. In the present embodiment, the monopolar manner has an output format in which a high-frequency current flows through an electrode set in which any one of the electrodes 21 to 23 and the counter electrode plate 25 are combined, and the bipolar manner has an output format in which a high-frequency current flows by using the electrodes 21 and 22 as an electrode set.

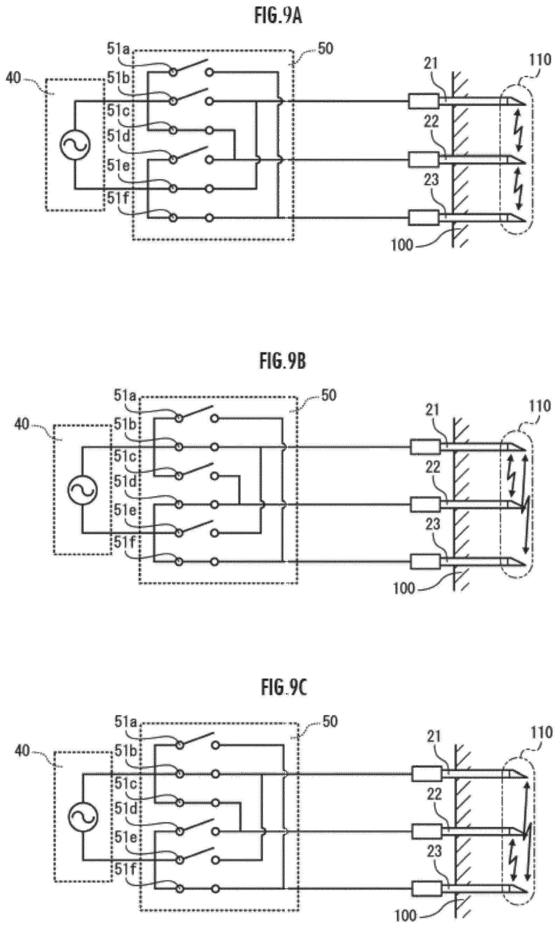

Hereinafter, the operation of the high-frequency treatment device 2 will be described by using a case where an output format is tripolar, as an example. FIGS. 9A to 9C are schematic views illustrating an operation state in a tripolar manner. In order to facilitate understanding, FIGS. 9A to 9C describe only the switches 51a to 51f necessary for description.

When local anesthesia for reducing pain due to overheating during a treatment and arrangement of the electrodes 21 to 23 are appropriately performed and the operation unit 16 accepts a tripolar manner selection operation by a user such as a doctor, the main control unit 80 controls the switching unit 50 to set a connection state between the output unit 40 and the electrodes 21 to 23 to be a connection state illustrated in FIG. 9A. Specifically, the main control unit 80 turns on the switch 51c, the switch 51e, and the switch 51f, and turns off the switch 51a, the switch 51b, and the switch 51d. Accordingly, with high-frequency power output by the output unit 40, a state is obtained in which a high-frequency current flows between the electrode 22, and the electrodes 21 and 23 in an electrode set configured with the three electrodes 21 to 23.

After that, when the operation unit 16 accepts a start operation of a treatment after the operation unit 16 receives an operation of selecting the high-frequency thermal coagulation method or the pulse high-frequency method and an input operation of a temperature setting value (for example, 80°C) and a treatment time (for example, 3 minutes) by the user (in a case where the pulse high-frequency method is selected, the main control unit 80 sets the temperature setting value to 42°C, so that only the treatment time is input), the main control unit 80 controls the output unit 40 to start an output of high-frequency power.

Further, the main control unit 80 controls the switching unit 50 to switch the connection state of the output unit 40 with the electrodes 21 to 23 between three states illustrated in FIGS. 9A to 9C in a specific cycle (0.1 seconds in the present embodiment). Specifically, the state illustrated in FIG. 9B is a state in which the switch 51b, the switch 51d, and the switch 51f are turned on and the switch 51a, the switch 51c, and the switch 51e are turned off, so a high-frequency current flows between the electrode 21, and the electrode 22 and the electrode 23. Further, the state illustrated in FIG. 9C is a state in which the switch 51b, the switch 51c, and the switch 51f are turned on and the switch 51a, the switch 51d, and the switch 51e are turned off, so a high-frequency current flows between the electrode 23, and the electrode 21 and the electrode 22.

That is, during the output of the high-frequency power, the main control unit 80 controls the switching unit 50 to switch any one of the electrodes 21 to 23 in order at a specific cycle so that a high-frequency current flows between the remaining two electrodes. Accordingly, a region between the electrodes 21 and 22 in the periphery of the electrodes 21 and 22, a region between the electrodes 22 and 23 in the periphery of the electrodes 22 and 23, and a region between the electrodes 23 and 21 in the periphery of the electrodes 23 and 21 are heated.

The main control unit 80 performs the output control of the first output unit 41 and the second output unit 42 in parallel with the switching control of the switching unit 50. The output control in the present embodiment has the same manner as the first embodiment except for the process of step S15 described above, and changes in the control state of the main control unit 80 and the temperature measurement value during the treatment also have the same manner as the first embodiment, so the description thereof will be omitted.

In the present embodiment, based on the temperature measurement value of the thermocouple 22c incorporated in the electrode 22 in the state illustrated in FIG. 9A, the temperature measurement value of the thermocouple 21c incorporated in the electrode 21 in the state illustrated in FIG. 9B, and the temperature measurement value of the thermocouple 23c incorporated in the electrode 23 in the state illustrated in FIG. 9C, the output control is performed by the main control unit 80 and the proportion control unit 230. That is, in the present embodiment, accuracy and stability of the output control are improved by referring to an ambient temperature of an electrode having the highest current density among the electrodes 21 to 23.

For example, it is possible to adopt various known shapes and arrangements. In addition, the high-frequency treatment devices 1 and 2 may be used for other purposes such as ablation of a tumor, for example.

For example, in the 1, the second output unit 42 may be omitted and high-frequency power may be output from the first output unit 41 to the electrodes 21 to 24 and the counter electrode plate 25, or the number of electrodes may be increased to 6 and a third output unit and a third temperature measurement unit may be provided at the same time. Further, in the high-frequency treatment device 2, the two output units 40 and the two temperature measurement units 60 may be provided, or the sub-control unit 90 that controls the switching unit 50, the temperature measurement unit 60, may be provided.

Further, the electrodes 21 to 24 may be, for example, disposed on a skin surface of a human body to heat a relatively shallow region under the skin. In addition, the number of electrodes constituting the electrode set is two or more.

### Description of Reference Numerals

1, 2 high-frequency treatment device
21 to 24 electrode
21c to 24c thermocouple
30 operation switch
40 output unit
41 first output unit
42 second output unit
60 temperature measurement unit
61 first temperature measurement unit
62 second temperature measurement unit
80 main control unit
100 treatment target
E1 first voltage value
E2 second voltage value

## Claims

1. A high-frequency treatment device (1, 2) comprising:
an output unit (40) configured to output high-frequency power;
a plurality of electrodes (21, 22, 23, 24) connected to the output unit (40) and configured to be disposed in or on a patient;
a temperature measurement unit (60) configured to measure an ambient temperature of at least one of the electrodes (21, 22, 23, 24);
a control unit (80) configured to perform normal output control of controlling the output of the output unit (40) so that a temperature measurement value measured by the temperature measurement unit (60) is equal to a control target value; and
an operation detection unit configured to detect an operation of the patient, **characterized in that**
the control unit (80) is configured to perform output reduction control of reducing the output of the output unit (40) below the output of the output unit (40) in the normal output control, based on the operation detection unit detecting the operation of the patient.

2. The high-frequency treatment (1, 2) device according to claim 1,
wherein the control unit (80) is configured to perform the output reduction control only while the operation detection unit detects the operation of the patient.

3. The high-frequency treatment device (1, 2) according to claim 1,
wherein the control unit (80) is configured to perform the output reduction control for a predetermined period, based on the operation detection unit detecting the operation of the patient.

4. The high-frequency treatment device (1, 2) according to any one of claims 1 to 3,
wherein in a case where the temperature measurement value is equal to or lower than a preset temperature reference value, the control unit (80) is configured to perform the normal output control even when the operation detection unit detects the operation of the patient.

5. The high-frequency treatment device (1, 2) according to any one of claims 1 to 4,
wherein the control unit (80)
is configured to set, in the normal output control, the control target value to a first target value and to control the output of the output unit (40) so that the temperature measurement value is equal to the control target value, and
is configured to set, in the output reduction control, the control target value to a second target value lower than the first target value and to control the output of the output unit (40) so that the temperature measurement value is equal to the control target value.

6. The high-frequency treatment device (1, 2) according to claim 5,
wherein in the output reduction control, the control unit (80) is configured to set the control target value to a temperature lower than the current temperature measurement value.

7. The high-frequency treatment device (1, 2) according to any one of claims 1 to 6,
wherein the operation detection unit is configured to change a first voltage value and a second voltage value to be input to the control unit (80) in a case where the operation of the patient is received, and
the control unit (80) is configured to determine whether or not the operation detection unit detects the operation of the patient based on a change of the first voltage value and the second voltage value.

## Patentansprüche

1. Hochfrequenz-Behandlungsgerät (1, 2), umfassend:
eine Ausgabeeinheit (40), die konfiguriert ist, Hochfrequenzleistung auszugeben,
mehrere Elektroden (21, 22, 23, 24), die mit der Ausgabeeinheit (40) verbunden sind und konfiguriert sind, in oder an einem Patienten angeordnet zu werden,
eine Temperaturmesseinheit (60), die konfiguriert ist, die Umgebungstemperatur mindestens einer der Elektroden (21, 22, 23, 24) zu messen,
eine Steuereinheit (80), die konfiguriert ist, eine Steuerung einer normalen Ausgabe des Steuerns der Ausgabe der Ausgabeeinheit (40) so durchzuführen, dass ein von der Temperaturmesseinheit (60) gemessener Temperaturmesswert einem Steuerungszielwert entspricht, und
eine Handlungserfassungseinheit, die konfiguriert ist, eine Handlung des Patienten zu erfassen,
**dadurch gekennzeichnet, dass**
die Steuereinheit (80) konfiguriert ist, eine Steuerung einer Ausgabeverringerung des Verringerns der Ausgabe der Ausgabeeinheit (40) auf unterhalb der Ausgabe der Ausgabeeinheit (40) bei der Steuerung der normalen Ausgabe basierend auf der die Handlung des Patienten erfassenden Handlungserfassungseinheit durchzuführen.

2. Hochfrequenz-Behandlungsvorrichtung (1, 2) gemäß Anspruch 1,
wobei die Steuereinheit (80) konfiguriert ist, die Steuerung einer Ausgabeverringerung nur durchzuführen, während die Handlungserfassungseinheit die Handlung des Patienten erfasst.

3. Hochfrequenz-Behandlungsvorrichtung (1, 2) gemäß Anspruch 1,
wobei die Steuereinheit (80) konfiguriert ist, die Steuerung einer Ausgabeverringerung über einen vorgegebenen Zeitraum basierend auf der die Handlung des Patienten erfassenden Handlungserfassungseinheit durchzuführen.

4. Hochfrequenz-Behandlungsvorrichtung (1, 2) gemäß einem der Ansprüche 1 bis 3, wobei in dem Fall, dass der Temperaturmesswert gleich oder niedriger als ein voreingestellter Temperatur-Referenzwert ist, die Steuereinheit (80) konfiguriert ist, die Steuerung der normalen Ausgabe durchzuführen, selbst wenn die Handlungserfassungseinheit die Handlung des Patienten erfasst.

5. Hochfrequenz-Behandlungsvorrichtung (1, 2) gemäß einem der Ansprüche 1 bis 4, wobei die Steuereinheit (80)
konfiguriert ist, bei der Steuerung der normalen Ausgabe den Steuerungszielwert auf einen ersten Zielwert einzustellen und die Ausgabe der Ausgabeeinheit (40) so zu steuern, dass der Temperaturmesswert dem Steuerungszielwert entspricht, und
konfiguriert ist, bei der Steuerung einer Ausgabeverringerung den Steuerungszielwert auf einen zweiten Zielwert, der niedriger als der erste Zielwert ist, einzustellen und die Ausgabe der Ausgabeeinheit (40) so zu steuern, dass der Temperaturmesswert dem Steuerungszielwert entspricht.

6. Hochfrequenz-Behandlungsvorrichtung (1, 2) gemäß Anspruch 5, wobei bei der Steuerung einer Ausgabeverringerung die Steuereinheit (80) konfiguriert ist, den Steuerungszielwert auf eine Temperatur, die niedriger als der aktuelle Temperaturmesswert ist, einzustellen.

7. Hochfrequenz-Behandlungsvorrichtung (1, 2) gemäß einem der Ansprüche 1 bis 6,
wobei die Handlungserfassungseinheit konfiguriert ist, einen in die Steuereinheit (80) einzugebenden ersten Spannungswert und zweiten Spannungswert in dem Fall, dass die Handlung des Patienten empfangen wird, zu ändern, und
die Steuereinheit (80) konfiguriert ist, zu erfassen, ob die Handlungserfassungseinheit die Handlung des Patienten basierend auf einer Änderung des ersten Spannungswerts und des zweiten Spannungswerts erfasst oder nicht erfasst.

## Revendications

1. Dispositif de traitement à haute fréquence (1, 2) comprenant :
une unité de sortie (40) configurée pour sortir une puissance à haute fréquence ;
une pluralité d'électrodes (21, 22, 23, 24) reliées à l'unité de sortie (40) et configurées pour être disposées dans ou sur un patient ;
une unité de mesure de température (60) configurée pour mesurer une température ambiante d'au moins l'une des électrodes (21, 22, 23, 24) ;
une unité de commande (80) configurée pour réaliser une commande de sortie normale consistant à commander la sortie de l'unité de sortie (40) de sorte qu'une valeur de mesure de température mesurée par l'unité de mesure de température (60) soit égale à une valeur cible de commande ; et
une unité de détection d'opération configurée pour détecter une opération du patient, **caractérisé en ce que**
l'unité de commande (80) est configurée pour réaliser une commande de réduction de sortie consistant à réduire la sortie de l'unité de sortie (40) en dessous de la sortie de l'unité de sortie (40) dans la commande de sortie normale, sur la base du fait que l'unité de détection d'opération détecte l'opération du patient.

2. Dispositif de traitement à haute fréquence (1, 2) selon la revendication 1, dans lequel l'unité de commande (80) est configurée pour réaliser la commande de réduction de sortie uniquement pendant que l'unité de détection d'opération détecte l'opération du patient.

3. Dispositif de traitement à haute fréquence (1, 2) selon la revendication 1, dans lequel l'unité de commande (80) est configurée pour réaliser la commande de réduction de sortie pendant une période prédéterminée, sur la base du fait que l'unité de détection d'opération détecte l'opération du patient.

4. Dispositif de traitement à haute fréquence (1, 2) selon l'une quelconque des revendications 1 à 3, dans lequel, dans un cas où la valeur de mesure de température est inférieure ou égale à une valeur de référence de température prédéfinie, l'unité de commande (80) est configurée pour réaliser la commande de sortie normale même lorsque l'unité de détection d'opération détecte l'opération du patient.

5. Dispositif de traitement à haute fréquence (1, 2) selon l'une quelconque des revendications 1 à 4,
dans lequel l'unité de commande (80)
est configurée pour définir, dans la commande de sortie normale, la valeur cible de commande à une première valeur cible et pour commander la sortie de l'unité de sortie (40) de sorte que la valeur de mesure de température soit égale à la valeur cible de commande, et
est configurée pour définir, dans la commande de réduction de sortie, la valeur cible de commande à une deuxième valeur cible inférieure à la première valeur cible et pour commander la sortie de l'unité de sortie (40) de sorte que la valeur de mesure de température soit égale à la valeur cible de commande.

6. Dispositif de traitement à haute fréquence (1, 2) selon la revendication 5, dans lequel, dans la commande de réduction de sortie, l'unité de commande (80) est configurée pour définir la valeur cible de commande à une température inférieure à la valeur de mesure de température actuelle.

7. Dispositif de traitement à haute fréquence (1, 2) selon l'une quelconque des revendications 1 à 6,
dans lequel l'unité de détection d'opération est configurée pour changer une première valeur de tension et une deuxième valeur de tension à entrer dans l'unité de commande (80) dans un cas où l'opération du patient est reçue, et
l'unité de commande (80) est configurée pour déterminer si l'unité de détection d'opération détecte ou non l'opération du patient sur la base d'un changement de la première valeur de tension et de la deuxième valeur de tension.
